# FASCICULE DE BREVET EUROPEEN

(11) **EP 1 352 049 B1**
(45) Date de publication et mention de la délivrance du brevet: **18.08.2010**
(21) Numéro de dépôt: 02710961.0
(22) Date de dépôt: 10.01.2002
(51) Int. Cl.: C12M 1/22, C12M 1/16, B65B 69/00

(54) **BOITE STERILE POUR LA CULTURE DE MICRO-ORGANISMES, ENSEMBLE ET DISTRIBUTEUR DE BOITES**
STERILE BOX ZUR KULTIVIERUNG VON MIKROORGANISMEN, BOXENSATZ UND BOXENSPENDER
STERILE BOX FOR MICRO-ORGANISM CULTURE, SET AND DISPENSER OF BOXES

(30) Priorité: 18.01.2001 FR 0100679
(43) Date de publication de la demande: 15.10.2003
(73) Titulaire: Lemonnier, Jean, 75006 Paris (FR); Maradan, Michel, 01460 Geovressiat (FR)
(72) Inventeur: Lemonnier, Jean, 75006 Paris (FR)
(74) Mandataire: Bezault, Jean
(86) Numéro de dépôt international: PCT/FR2002/000083
(87) Numéro de publication internationale: WO 2002/057405

(56) Documents cités:
- EP-A- 0 447 893
- FR-A- 2 436 084
- FR-A- 2 486 915
- US-A- 3 816 264
- US-A- 4 709 819

## Description

L'invention concerne une boîte stérile pour la culture de micro-organismes.

Elle concerne plus particulièrement une boîte constituée d'un récipient fermé par un couvercle et apte à contenir un milieu de culture, ce récipient comportant un fond et des parois raccordées au fond.

Des boîtes de ce type, fréquemment appelées boîtes de Pétri, sont généralement en matière plastique injectée. Elles sont, de préférence, transparentes afin de permettre l'examen visuel du milieu de culture, par exemple un milieu gélosé. Ces boîtes ne sont pas étanches. Elles sont conditionnées dans un emballage étanche et stérile qui contient plusieurs boîtes. Dès l'ouverture de l'emballage, la stérilité des boîtes n'est plus assurée. FR 2436084 décrit des boîtes de Pétri emballées dans un ensemble étanche et en matière synthétique.

Par ailleurs, l'ouverture de l'emballage des boîtes, qui de ce fait se retrouvent à l'air libre, réduit significativement leur délai de péremption. Il est donc nécessaire d'utiliser rapidement l'ensemble des boîtes d'un même emballage. En outre, lors de l'analyse microbiologique sur membrane filtrante, il est nécessaire de transférer et de déposer sur la surface du milieu de culture une membrane ayant retenu d'éventuels germes. Cette opération doit se faire dans de bonnes conditions d'asepsie. On procède, de préférence, à cette opération délicate dans un laboratoire de contrôle où l'air est propre, ou encore en manipulant sous une hotte à flux laminaire où l'air est filtré et donc débarrassé d'une éventuelle contamination microbienne qui pourrait modifier les résultats de l'analyse.

D'une main, l'opérateur lève le couvercle de la boîte stérile (boîte de Pétri) qui repose généralement sur une paillasse. De son autre main, il transfère la membrane filtrante à l'aide de pincettes du point de filtration jusqu'au récipient de la boîte dans laquelle il la dépose. Il referme ensuite la boîte et la place à l'envers dans un incubateur.

L'éloignement de la boîte de Pétri du point de filtration de l'échantillon n'est pas favorable et peut être la source de contaminations exogènes en cas de qualité insuffisante de l'air.

La présente invention a précisément pour objet de proposer une boîte stérile pour la culture de micro-organismes qui remédie à ces inconvénients. En particulier, elle propose des boîtes prêtes à l'emploi, totalement étanches, dont la durée individuelle de péremption d'un même lot de fabrication est homogène.

Ce résultat est obtenu, conformément à l'invention, par le fait que le couvercle est posé sur le récipient et comporte une zone périphérique de scellement constituée par un méplat en retrait par rapport au fond, formant assise, du récipient afin que le méplat ne touche pas la surface sur laquelle repose la boîte, et par le fait qu'un opercule de forme concave est fixé de manière étanche sur la zone de scellement du couvercle, de manière à fermer hermétiquement la boîte en enveloppant le récipient, et à ne pas découvrir le milieu de culture lorsque l'opercule est ôté.

Grâce à cette caractéristique, chaque boîte prise individuellement est étanche. Les boîtes peuvent être conditionnées dans un emballage qui peut être ouvert sans rupture de l'étanchéité de chacune d'elles. En conséquence, il n'est pas nécessaire d'utiliser l'ensemble des boîtes d'un emballage en même temps. Chaque boîte garde sa durée de péremption propre. Il est possible d'ôter l'opercule dans un environnement inapproprié, sans découvrir le milieu de culture et, de ce fait, sans rompre instantanément la stérilité de la chambre de culture située entre la surface du milieu de culture et le fond du couvercle.

De préférence, l'opercule est transparent.

L'opercule est formé en une matière plastique semi-rigide, et sa forme concave est avantageusement thermoformée.

Selon un mode de réalisation particulier, le couvercle de la boîte comporte un rebord flexible entourant les parois du récipient, ce rebord pouvant être déformé pour pincer les parois du récipient.

De préférence, le rebord flexible comporte une partie en saillie apte à coopérer avec une partie complémentaire du récipient lorsque le rebord est déformé, de manière à retenir le couvercle sur le récipient.

Grâce à ces caractéristiques, la préhension et le serrage du diamètre extérieur du couvercle entre le pouce et l'index d'une main permettent de maintenir le récipient fermement en place dans le couvercle par le pinçage du rebord flexible du récipient et par l'engagement de la partie en saillie du rebord flexible dans la partie complémentaire du récipient. Cette disposition empêche le récipient de s'échapper lorsque la boîte est tenue à l'endroit.

Dans un mode de réalisation préféré, le récipient comporte un rebord en forme de L inversé qui entoure au moins partiellement les parois du récipient, la paroi flexible du couvercle étant apte à pincer le rebord en forme de L inversé du récipient sans provoquer de déformation des parois du récipient.

Ainsi, le milieu de culture, par exemple gélosé, contenu dans le récipient n'est pas perturbé par une déformation lorsque l'on pince la boîte entre le pouce et l'index.

Dans un mode de réalisation préféré, la boîte de l'invention comporte des moyens d'articulation du couvercle sur le récipient, de manière à permettre l'ouverture et la fermeture dans l'espace de la boîte d'une seule main.

Dans un mode particulier de réalisation, les moyens d'articulation sont constitués par au moins un tenon prévu sur le couvercle et par au moins une fente complémentaire prévue dans le récipient, fente dans laquelle s'engage le tenon.

Il en résulte également la possibilité d'ouvrir ou de fermer la boîte axialement par un mouvement vertical du couvercle. L'invention ne se limite pas à ce système d'articulation du couvercle sur le récipient. Par exemple, le couvercle pourrait être articulé par une charnière, ou par tout autre moyen analogue.

Grâce à l'articulation du couvercle sur le récipient, la boîte peut être ouverte ou fermée instantanément d'une seule main. L'index et/ou le majeur soutiennent le dessous du récipient. En reculant ou en avançant légèrement le pouce en appui sur le couvercle, on ouvre ou on ferme la boîte instantanément.

Dans un mode de réalisation particulier, le récipient comporte une couronne extérieure et le couvercle comporte une paroi intérieure et une paroi extérieure, le tenon étant prévu sur la paroi intérieure du couvercle, et la fente étant prévue dans la couronne du récipient.

Avantageusement, la fente de la couronne du récipient comporte deux surfaces de butée pour limiter l'ouverture du couvercle. Ainsi, l'angle d'ouverture du couvercle est constant et limité, d'une part, par le contact de la paroi circulaire intérieure du couvercle qui vient en appui sur la partie intérieure de la couronne du récipient et, d'autre part, par le fait que le tenon vient en butée sur la partie extérieure, avantageusement chanfreinée, de la fente pratiquée sous le fond du récipient. Cette double butée limite l'angle d'ouverture du couvercle à 60 environ.

De préférence, le couvercle est muni d'au moins un ergot de fonctionnement qui facilite l'ouverture du couvercle.

Chaque boîte peut comporter un opercule individuel. L'invention concerne également un ensemble de boîtes constitué d'une bande et d'une série de boîtes selon l'invention, disposées les unes derrière les autres sur la bande, chaque boîte étant fixée sur la bande par sa zone de scellement, la bande jouant le rôle d'un opercule pour chacune des boîtes.

Les boîtes peuvent être disposées sur une seule face de la bande formant l'opercule, ou bien les boîtes peuvent être disposées alternativement sur une face et sur l'autre de la bande. Cette dernière disposition permet de réduire l'espace entre chaque boîte dans le cas d'un empilage des boîtes dans une position identique (à l'endroit ou à l'envers) lorsque la bande est pliée en accordéon.

Enfin, l'invention concerne un distributeur de boîtes conformes à l'invention, ces boîtes faisant partie d'un ensemble de boîtes fixées sur une bande constituant un opercule pour chaque boîte. Le distributeur comporte un magasin de stockage de la bande ; des moyens d'entraînement de la bande ; un moyen de séparation pour séparer une boîte de la bande ; un plateau de réception de la boîte après sa séparation de la bande.

Afin de guider les boîtes avant leur arrivée sur les moyens de séparation, le distributeur comporte avantageusement un guide qui présente les boîtes successives de la bande avec un angle d'inclinaison par rapport au sens d'avance de la bande.

Lorsque les boîtes sont disposées en vis-à-vis sur les deux faces de la bande, le guide pivote entre deux positions inclinées symétriquement par rapport au sens d'avance de la bande, le guide passant d'une position à l'autre de ces positions en fonction de la face sur laquelle la boîte est fixée.

Ce distributeur permet une distribution automatisée des boîtes sans rupture de la bande, ce qui permet l'enroulement de cette dernière et sa récupération. On évite ainsi les déchets d'opercules individuels.

D'autres caractéristiques et avantages de l'invention apparaîtront encore à la lecture de la description qui suit d'un exemple de réalisation donné en référence aux figures annexées. Sur ces figures :
- la Figure 1 est une vue en coupe d'une boîte selon un premier mode de réalisation de l'invention ;
- la Figure 2 est une vue en coupe de la boîte représentée sur la Figure 1 en position de serrage du couvercle ;
- la Figure 3 est une vue de dessus de la boîte des Figures 1 et 2 ;
- la Figure 4 est une vue en coupe d'une boîte selon un second mode de réalisation de l'invention, dont le couvercle est en position fermée ;
- la Figure 5 est un détail, représenté à échelle agrandie, de la figure 4, dont la paroi extérieure du couvercle est serrée sur la couronne extérieure du récipient ;
- la Figure 6 est une autre vue en coupe analogue à celle de la Figure 4 ;
- la Figure 7 est une vue en coupe de la boîte représentée sur la Figure 4 montrant le couvercle en position ouverte ;
- la Figure 8 est un détail, représenté à échelle agrandie, de la figure 7 ;
- la Figure 9 est une vue de dessus de la boîte des Figures 4 à 8 ;
- la Figure 10 montre la position des doigts d'un opérateur se servant d'une boîte conforme aux Figures 4 à 9, dont le couvercle est ouvert ;
- la Figure 11 représente un ensemble de boîtes selon l'invention fixées sur une seule face d'une bande formant opercule pour chaque boîte ;
- la Figure 12 représente un ensemble de boîtes selon l'invention fixées alternativement sur les deux faces d'une bande formant opercule pour chaque boîte ;
- la Figure 13 représente l'ensemble de boîtes de la Figure 12 après pliage de la bande en accordéon pour former un empilage de boîtes ; et
- la Figure 14 représente un distributeur de boîtes conformes à la présente invention.

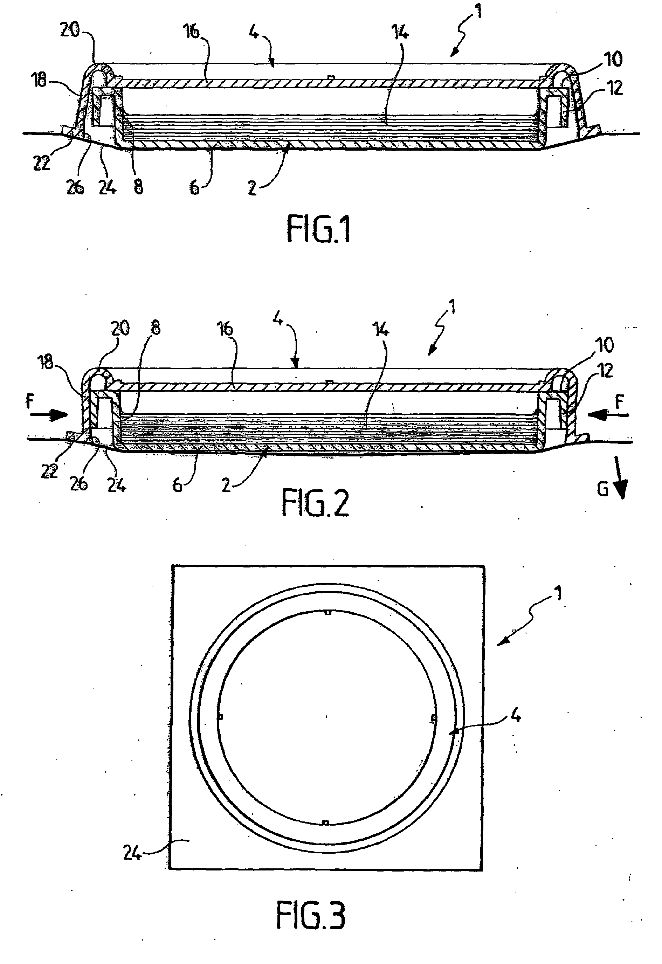

On a représenté sur les Figures 1 et 2 des vues en coupe d'un premier mode de réalisation particulier d'une boîte stérile 1 conforme à l'invention. La boîte 1 est constituée d'un récipient 2 surmonté d'un couvercle 4. Le récipient 2 comporte un fond 6 qui forme assise et auquel sont raccordées des parois verticales 8. Dans l'exemple, le fond 6 est circulaire et les parois 8 sont cylindriques circulaires. Les parois 8 se terminent par une collerette horizontale 10, elles-mêmes prolongées par un retour 12 sensiblement parallèle aux parois 8. Le retour 12 forme avec la collerette 10 et les parois 8 un rebord en forme de L inversé.

Le récipient 2 est apte à contenir un milieu de culture 14. Ce milieu est, par exemple, un milieu de culture gélosé coulé dans le récipient 2.

Le couvercle 4 comporte une paroi 16 de forme circulaire qui ferme le récipient 2 en reposant sur la collerette horizontale 10. Le couvercle 4 comporte un rebord flexible 18 relié au pourtour de la paroi 16 par une partie arrondie 20 destinée à favoriser la flexibilité du rebord 18.

Le couvercle 4 comporte, en outre, une zone de scellement périphérique. Dans l'exemple représenté, cette zone est constituée par un méplat 22 situé à l'extrémité du rebord flexible 18 et entourant le récipient 2. Comme on peut le constater sur la Figure 1, le méplat 22 est situé en retrait par rapport au fond 6 du récipient 2. Un opercule semi-rigide 24 (Figures 1 à 3) est fixé au méplat 22, par exemple, thermoscellé, collé ou fixé par tout autre moyen au méplat 22.

La boîte est fermée par le dessous, et l'opercule 24 maintient la stérilité de tout l'intérieur de la boîte en enveloppant entièrement le récipient 2 du côté du fond 6 de ce dernier. En d'autres termes, le récipient est entièrement contenu dans le volume intérieur délimité par le couvercle 4 et l'opercule 24. En outre, étant donné que le méplat 22 est situé en retrait par rapport au fond 6 du récipient 2, l'opercule 24, vu de l'extérieur, présente une forme concave. Une légère pré-tension de cet opercule permet de maintenir le couvercle 4 en appui sur la collerette 10 du récipient 2.

La boîte représentée sur les Figures 1 à 3 comporte une protection individuelle de stérilité assurée par l'opercule semi-rigide 24. Chaque boîte comporte son propre opercule, ici de forme carrée, de telle sorte que les boites faisant partie d'un même emballage ne perdent leur stérilité que si l'opercule 24 est retiré.

La surface du milieu de culture 14 est visible à travers une seule paroi transparente, à savoir la paroi 16 du couvercle 4. La visibilité est donc optimale, ce qui permet un examen de la surface du milieu de culture gélosé 14 avant le pelage de l'opercule 24.

Dans l'exemple de réalisation décrit, la boîte est circulaire et son diamètre est de 70 mm environ, ce qui permet de la tenir facilement entre le pouce et les autres doigts de la main. La boîte peut être également ovale, polygonale ou présenter encore une autre forme.

L'opercule 24 est formé d'une matière plastique et est transparent.

Comme on peut le constater sur la Figure 2, le rebord flexible 18 permet de maintenir le récipient 2 fermement en place dans le couvercle 4 par le pinçage du diamètre extérieur du retour 12. Dans l'exemple représenté, l'intérieur du rebord flexible 18 comporte une partie en saillie 26 apte à s'engager sous le rebord 12 du récipient 2 lorsque le rebord flexible 18 est pincé entre le pouce et l'index d'une main, comme schématisé par les flèches F. Dans l'exemple représenté, la partie en saillie est constituée par une contre-dépouille 26 qui assure le verrouillage du couvercle 4 sur le récipient 2. La contre-dépouille empêche le récipient de s'échapper lorsque la boîte est tenue à l'endroit, la paroi extérieure du couvercle maintenue serrée.

Par ailleurs, le rebord flexible 18 prend appui sur le retour 12. Cela permet de pincer le couvercle sans provoquer une déformation des parois verticales 8 et, par suite, sans déformer le milieu de culture 14. Pour ôter l'opercule, on exerce une traction sur une extrémité de celui-ci, comme montré par la flèche G de la figure 2, puis on pèle ce dernier.

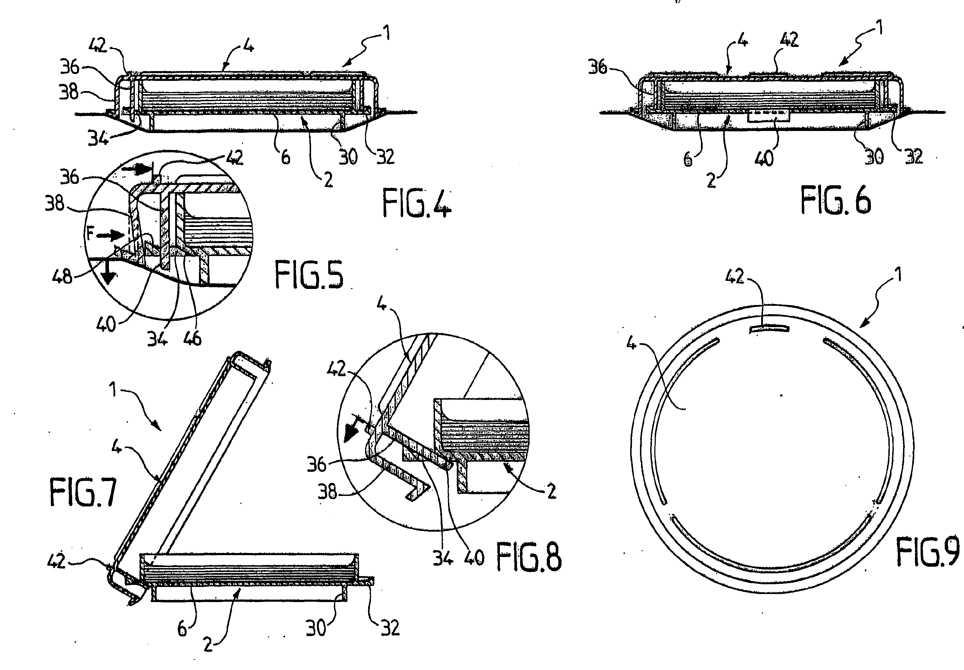

On a représenté sur les Figures 4 à 10 un second mode particulier de réalisation d'une boîte stérile pour la culture de micro-organismes en vue d'effectuer des analyses microbiologiques. Ce mode de réalisation se distingue par le fait qu'il comporte des moyens d'articulation du couvercle sur le récipient, de manière à permettre l'ouverture et la fermeture de la boîte d'une seule main. Dans l'exemple particulier représenté, le fond 6 du récipient 2 est muni d'une nervure circulaire d'assise 30, constituée, par exemple, de trois parties disposées à 120° l'une de l'autre, sur lesquelles le récipient repose lorsqu'il est disposé à l'endroit, par exemple sur une paillasse. Le fond 6 est prolongé à sa périphérie par une couronne 32 qui présente, de préférence, la forme d'une coupelle creuse, de manière à faciliter le centrage du couvercle par rapport au récipient. Une fente 34 (voir en particulier les Figures 5 et 8), destinée à recevoir un tenon faisant partie du couvercle, est prévue dans la couronne 32. On peut également prévoir plusieurs fentes, par exemple trois, afin de permettre la mise en place du couvercle dans des positions différentes sur le récipient.

Le couvercle 4 comporte une paroi intérieure circulaire 36 et une paroi extérieure 38, également circulaire. Un tenon 40 est prévu sur la paroi intérieure 36 du couvercle 4. Le couvercle 4 est également muni d'un ergot de fonctionnement 42 sur lequel le pouce d'une main vient en appui afin de permettre l'ouverture et la fermeture du couvercle 4.

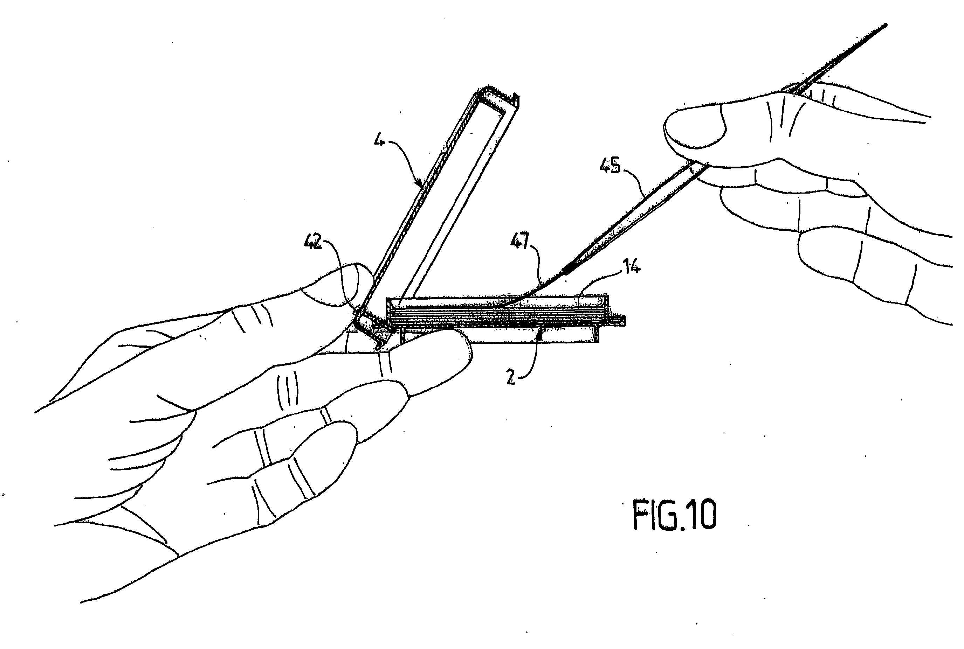

La boîte représentée sur les Figures 4 à 9 peut être maintenue dans l'espace d'une seule main, par exemple de la main gauche d'un opérateur, comme montré à la figure 10. Elle peut être ouverte ou fermée instantanément de cette même main. La fente 34 comporte des surfaces de butée pour limiter le déplacement du tenon 40 et, par suite, l'angle d'ouverture du couvercle 4. Dans l'exemple représenté, une première surface de butée est constituée par un chanfrein 46 formé vers l'intérieur de la fente 34, et d'autre part par un chanfrein circulaire 48 (figure 5). Cette double butée limite l'angle d'ouverture du couvercle 4 à 60° environ.

Grâce à cette caractéristique, la boîte peut être placée près d'un point de filtration d'un échantillon à analyser, en étant maintenue à l'aide d'une seule main. On peut ainsi, de l'autre main, saisir et transférer une membrane 47 à l'aide de pincettes 45, puis la déposer sur le milieu de culture 14 contenu dans la boîte (Figure 10). La boîte est ouverte rapidement, seulement lorsque cela est nécessaire, pour déposer la membrane. Cette dernière n'est déplacée que de quelques centimètres dans un minimum de temps. Le couvercle s'ouvre partiellement, et seulement de l'angle nécessaire pour permettre de déposer la membrane sur le milieu de culture.

Ainsi, le milieu de culture reste à peu près constamment couvert par le couvercle, ce qui lui évite d'être trop exposé à l'environnement. En outre, le milieu de culture est à l'abri des doigts qui maintiennent la boîte car ceux-ci sont situés derrière le couvercle qui fait office d'écran ou de bouclier de protection. Le risque d'une contamination accidentelle due à la main qui tient la boîte est ainsi éliminé. Dans le cas de manipulations sous une hotte à flux laminaire horizontal, il est à noter que la boîte, placée près du point de filtration, est située dans la trajectoire du flux. Ainsi, la dépose de la membrane sur le milieu de culture est faite dans les meilleures conditions d'asepsie, où aucun obstacle ne masque le flux. Enfin, le très faible déplacement de la membrane lors du test permet d'éviter au maximum le ruissellement provenant du dessous de la membrane et, ainsi, de maintenir un film liquide qui favorise son contact avec le milieu de culture 14.

La paroi extérieure 38 constitue un rebord flexible qui peut être pincé entre les doigts de la main, par exemple le pouce et l'index selon le sens des flèches F, de manière à venir serrer le diamètre extérieur de la couronne 32. D'une manière identique à ce qui a été décrit en référence aux Figures 1 et 2, le couvercle 4 est ainsi verrouillé sur le récipient 2 par le pinçage du diamètre extérieur de la paroi 38, ce qui empêche le récipient de s'échapper lorsque la boîte est tenue à l'endroit.

L'analyse microbiologique sur une membrane filtrante au moyen d'une boîte stérile de l'invention s'effectue, par conséquent, de la manière suivante. Après filtration de l'échantillon à analyser, on saisit de la main gauche (cas d'une personne droitière) la boîte par son diamètre entre le pouce et l'index et on la serre de manière que le couvercle retienne le récipient par serrage, comme on l'a décrit précédemment. On ôte l'entonnoir de filtration de son support et, de la main droite, on pèle l'opercule en le tirant vers le bas. La boîte est prête à être utilisée.

Il est également possible de peler l'opercule de la boîte retournée et maintenue par le diamètre extérieur du couvercle. Dans ce cas, il n'est pas nécessaire de pincer le couvercle entre le pouce et l'index puisque le récipient est en appui dans le fond du couvercle et ne risque pas de tomber. On prend de la main gauche la boîte par son épaisseur entre le pouce et l'index, le pouce placé sur l'ergot situé sur le couvercle, et l'index situé sous la boîte. On amène la boîte près du support de filtration, on saisit de la main droite la membrane à l'aide de pincettes, on recule légèrement le pouce de la main gauche, de telle sorte que la boîte s'ouvre. On dépose la membrane sur la gélose de la main droite, et on ramène le pouce de la main gauche vers l'avant, de telle sorte que le couvercle se referme instantanément sur le récipient. On place la boîte ainsi retournée dans l'incubateur et, de manière classique, après le temps nécessaire pour la croissance des éventuelles colonies microbiennes ou bactériennes, on effectue le dénombrement et l'identification le cas échéant.

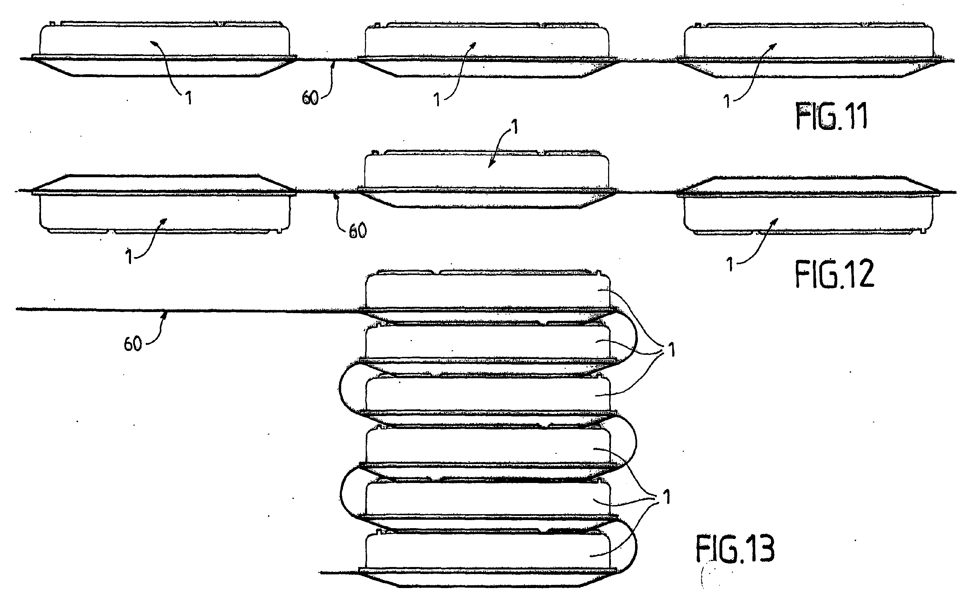

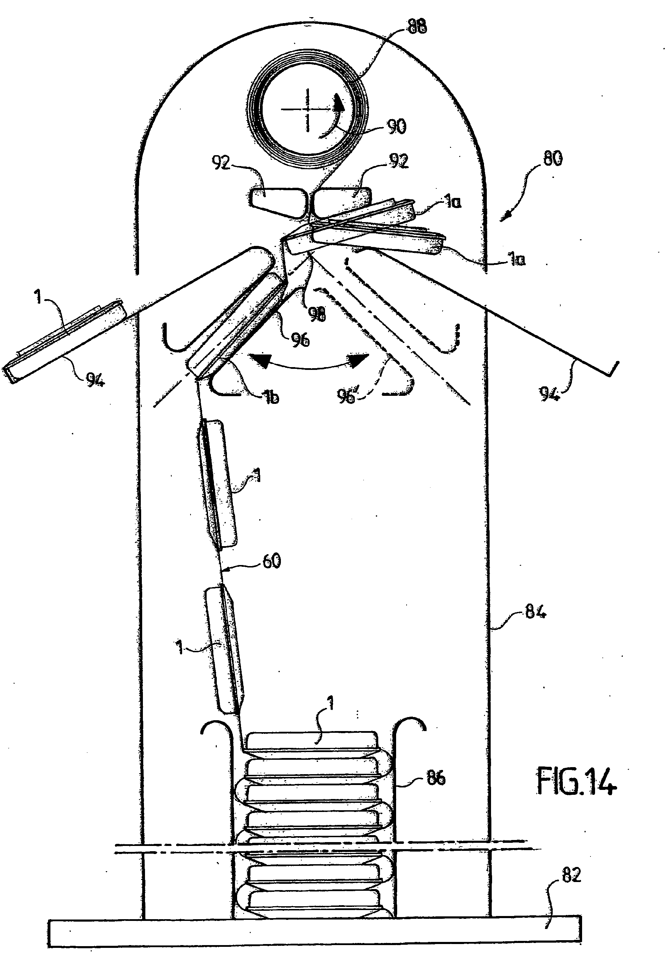

Une boîte de l'invention peut comporter un opercule individuel. Il est également possible de disposer une série de boîtes sur un opercule commun constitué par une bande 60, comme représenté sur la Figure 11. Les boîtes 1 sont scellées les unes derrière les autres sur la bande de protection étanche 60. Les boîtes peuvent être scellées sur une même face de la bande 60, comme représenté sur la Figure 11. Elles peuvent également, comme représenté sur la Figure 12, être scellées alternativement sur une face et sur l'autre de la bande. Cette disposition permet le pliage en accordéon de la bande et l'empilage des boîtes dans un même sens, par exemple à l'endroit, en réduisant au minimum la longueur de la bande 60. Un tel empilage des boîtes, avec la bande en accordéon, est représenté sur la Figure 13. Un tel conditionnement offre l'avantage de pouvoir distribuer les boîtes 1 à l'aide d'un appareil distributeur adapté, tel que représenté sur la Figure 14.

Le distributeur 80 représenté sur la Figure 14 comporte un socle 82 sur lequel est fixé un boîtier 84 vertical. Sur la face avant du boîtier 84, on trouve un magasin de stockage 86 dans lequel est placée une bande 60 semblable à celle qui a été décrite en référence aux Figures 12 et 13. Les boîtes stériles 1 sont disposées les unes derrière les autres sur la bande 60, de part et d'autre des faces de cette bande.

A la partie supérieure du boîtier, on trouve un rouleau 88 d'entraînement de la bande qui tourne dans le sens indiqué par la flèche 90. La bande passe entre deux guides de séparation 92. Au moment où une boîte 1 vient en contact avec les guides de séparation 92, la force de traction exercée sur la bande sépare l'opercule de la boîte. Cette dernière pivote, puis glisse sur un plateau de réception 94.

Afin de faciliter la séparation de la boîte et de la bande, il est préférable que la boîte se présente sur les guides de séparation avec un certain angle d'incidence, par exemple 45°. C'est pourquoi l'invention comporte un guide tubulaire dans lequel les boîtes passent les unes après les autres avant d'atteindre les guides de séparation 92. En outre, étant donné que, dans le mode de réalisation représenté sur la Figure 14, les boîtes sont placées de part et d'autre de la bande, le guide 96 est pivotant. Il pivote d'un angle sensiblement égal à 90° autour d'un axe 98. Sur la Figure 14, le guide 96 a été représenté dans la position qu'il occupe pour présenter la boîte désignée par la référence 1a avec un angle d'incidence convenable par rapport aux deux guides 92. Lorsque la boîte suivante, désignée par la référence 1b, se présentera face au guide de séparation 92, le guide pivotant sera dans la position représentée en traits pointillés et désignée par la référence 96'. Ainsi, le guide pivotant 96 occupe alternativement des positions symétriques par rapport au plan de symétrie verticale du bâti 84 qui correspond sensiblement au sens d'avance de la bande 60. Après séparation, les boîtes sont reçues dans les plateaux symétriques 94 en position inversée. Elles sont alors prêtes à l'emploi.

## Revendications

1. Boîte stérile pour la culture de micro-organismes, constituée d'un récipient (2) fermé par un couvercle (4) et apte à contenir un milieu de culture (14), ce récipient comportant un fond (6) et des parois (8) raccordées au fond (6), **caractérisée en ce que** le couvercle (4) est posé sur le récipient (2) et comporte une zone périphérique de scellement (22) constituée par un méplat en retrait par rapport au fond (6), formant assise, du récipient (2) afin que le méplat ne touche pas la surface sur laquelle repose la boîte, et **en ce qu'**un opercule (24) de forme concave est fixé de manière étanche sur la zone de scellement (22) du couvercle (4), de manière à fermer hermétiquement la boîte (1) en enveloppant le récipient (2), et à ne pas découvrir le milieu de culture (14) lorsque l'opercule (24) est ôté.

2. Boîte selon la revendication 1, **caractérisée en ce que** l'opercule (24) est transparent.

3. Boîte selon l'une des revendications 1 et 2, **caractérisée en ce que** le couvercle (4) comporte un rebord flexible (18) entourant les parois (8) du récipient (2), ce rebord (18) pouvant être déformé pour pincer les parois (8) du récipient.

4. Boîte selon la revendication 3, **caractérisée en ce que** le rebord flexible (18) comporte une partie en saillie (26) apte à coopérer avec une partie complémentaire du récipient (2) lorsque le rebord (18) est déformé, de manière à retenir le couvercle (4) sur le récipient (2).

5. Boîte selon la revendication 3 ou 4, **caractérisée en ce que** le récipient (2) comporte un rebord (10, 12) en forme de L inversé qui entoure au moins partiellement les parois (8) du récipient, la paroi flexible (18) du couvercle (4) étant apte à pincer le rebord (10, 12) en forme de L du récipient sans provoquer de déformation des parois (8) du récipient (2).

6. Boîte selon l'une des revendications 1 à 5, **caractérisée en ce qu'**elle comporte des moyens d'articulation du couvercle (4) sur le récipient (2), de manière à permettre l'ouverture et la fermeture dans l'espace de la boîte (1) d'une seule main.

7. Boîte selon la revendication 6, **caractérisée en ce que** les moyens d'articulation sont constitués par au moins un tenon (40) prévu sur le couvercle (4) et par au moins une fente complémentaire (34) prévue dans le récipient (2), fente (34) dans laquelle s'engage le tenon, ce qui permet également d'ouvrir ou de fermer la boîte par un mouvement vertical du couvercle.

8. Boîte selon la revendication 7, **caractérisée en ce que** le récipient (2) comporte une couronne extérieure (32) et **en ce que** le couvercle (4) comporte une paroi intérieure (36) et une paroi extérieure (38), le tenon (40) étant prévu sur la paroi intérieure (36) du couvercle (4) et la fente (34) étant prévue dans la couronne (32) du récipient (2).

9. Boîte selon la revendication 7 ou 8, **caractérisée en ce que** la fente (34) de la couronne (32) du récipient (2) comporte deux surfaces (46, 48) de butée pour limiter l'ouverture du couvercle (4).

10. Boîte selon l'une des revendications 6 à 9, **caractérisée en ce que** le couvercle (4) est muni d'un ergot de fonctionnement (42) qui facilite son ouverture.

11. Ensemble de boîtes (1), **caractérisé en ce qu'**il est constitué d'une bande (60) et d'une série de boîtes (1) selon l'une des revendications 1 à 10, disposées l'une derrière l'autre sur la bande (60), chaque boîte (1) étant fixée sur la bande (60) par sa zone de scellement (22), la bande (60) jouant le rôle d'un opercule (24) pour chaque boîte (1).

12. Ensemble de boîtes selon la revendication 11, **caractérisé en ce que** les boîtes (1) sont disposées sur une seule face de la bande (60).

13. Ensemble de boîtes selon la revendication 11, **caractérisé en ce que** les boîtes (1) sont disposées alternativement sur une face et sur l'autre de la bande (60).

14. Distributeur pour la distribution de boîtes selon l'une des revendications 1 à 10, ces boîtes faisant partie d'un ensemble de boîtes selon l'une des revendications 11 à 13, **caractérisé en ce qu'**il comporte un magasin de stockage (86) de la bande (60) ; un moyen d'entraînement (88) de la bande ; un moyen de séparation (92) pour séparer une boîte (1) de la bande (60) ; au moins un plateau de réception (94) des boîtes (1) après leur séparation de la bande (60).

15. Distributeur selon la revendication 14, **caractérisé en ce** en ce que la bande est conforme à la revendication 13, en qu'il comporte un guide (96) qui présente les boîtes (1) successives de la bande (60) avec un angle d'inclinaison par rapport au sens d'avance de la bande (60), et en ce que le guide (96) pivote entre deux positions inclinées symétriquement par rapport au sens d'avance de la bande (60), le guide (96) prenant l'une ou l'autre de ces positions en fonction de la face de la bande sur laquelle la boîte est fixée.

## Claims

1. Sterile box for the culture of micro-organisms, consisting of a container (2) closed with a lid (4) and capable of containing a culture medium (14), the container forming a bottom (6) and walls (8) joined to the bottom (6), **characterised in that** the lid (4) is placed on the container (2) and comprises a peripheral sealing region (22) formed by a plane face set back from the bottom (6), forming a seat, of the container (2) in order that the plane face does not touch the surface on which the box rests, and **in that** a protective cover (24) of concave form is fixed in a sealing-tight manner to the sealing region (22) of the lid (4), so as to seal the box (1) hermetically by enveloping the container (2), and so as not to expose the culture medium (14) when the protective cover (24) is removed.

2. Box according to claim 1, **characterised in that** the protective cover (24) is transparent.

3. Box according to either of claims 1 or 2, **characterised in that** the lid (4) comprises a flexible rim (18) surrounding the walls (8) of the container (2), this rim (18) being deformable so as to grip the walls (8) of the container.

4. Box according to claim 3, **characterised in that** the flexible rim (18) comprises a projecting part (26) capable of cooperating with a complementary part of the container (2) when the rim (18) is deformed, so as to keep the lid (4) on the container (2).

5. Box according to claim 3 or 4, **characterised in that** the container (2) comprises an inverted L-shaped rim (10, 12) which surrounds the walls (8) of the container at least partially, the flexible wall (18) of the lid (4) being capable of gripping the L-shaped rim (10, 12) of the container without causing any deformation of the walls (8) of the container (2).

6. Box according to one of claims 1 to 5, **characterised in that** it comprises means of articulating the lid (4) to the container (2), so as to permit opening and closing within the space of the box (1) with one hand.

7. Box according to claim 6, **characterised in that** the means of articulation consist of at least one pin (40) provided on the lid (4) and by at least one complementary slot (34) provided in the container (2), in which slot (34) the pin (40) engages, which also permits opening and closing of the box by a vertical movement of the lid.

8. Box according to claim 7, **characterised in that** the container (2) comprises an outer collar (32) and **in that** the lid (4) comprises an inner wall (36) and an outer wall (38), the pin (40) being provided on the inner wall (36) of the lid (4) and the slot (34) being provided in the collar (32) of the container (2).

9. Box according to claim 7 or 8, **characterised in that** the slot (34) of the collar (32) of the container (2) comprises two stop faces (46, 48) in order to limit opening of the lid (4).

10. Box according to one of claims 6 to 9, **characterised in that** the lid (4) is equipped with an operating tappet (42) which facilitates its opening.

11. Set of boxes (1), **characterised in that** it consists of a strip (60) and of a series of boxes (1) according to one of claims 1 to 10, disposed one behind another on the strip (60), each box (1) being fixed to the strip (60) by its sealing region (22), the strip (60) acting as a protective cover (24) for each box (1).

12. Set of boxes according to claim 11, **characterised in that** the boxes (1) are disposed on only one face of the strip (60).

13. Set of boxes according to claim 11, **characterised in that** the boxes (1) are disposed alternately on either side of the strip (60).

14. Dispenser for dispensing boxes according to one of claims 1 to 10, these boxes forming part of a set of boxes according to one of claims 11 to 13, **characterised in that** it comprises a storage magazine (86) of the strip (60); a means (88) of driving the strip; a separating means (92) for separating one box (1) from the strip (60); and at least one receiving plate (94) for the boxes (1) after their separation from the strip (60).

15. Dispenser according to claim 14, **characterised in that** the strip is in accordance with claim 13, **in that** it comprises a guide (96) which presents the successive boxes (1) of the strip (60) at an angle of incline relative to the feed direction of the strip (60), and **in that** the guide (96) pivots between two positions inclined symmetrically relative to the feed direction of the strip (60), the guide (96) adopting one of these two positions according to the side of the strip to which the box is fixed.

## Patentansprüche

1. Sterile Box zur Kultivierung von Mikroorganismen, gebildet aus einem Behälter (2), der von einem Deckel (4) geschlossen ist und in der Lage ist, ein Kulturmilieu (14) zu enthalten, wobei der Behälter einen Boden (6) und Seitenwände (8) aufweist, die mit dem Boden (6) verbunden sind, **dadurch gekennzeichnet, dass** der Deckel (4) auf den Behälter (2) gesetzt ist und einen Umfangsanschlussflansch (22) aufweist, der von einer Abflachung gebildet wird, die gegenüber dem Boden (6), der einen Sitz des Behälters (2) bildet, zurückgezogen ist, damit die Abflachung nicht die Oberfläche berührt, auf der das Gehäuse ruht, und dass ein Verschlussorgan (24) von konkaver Form auf dichtende Weise an der Abflanschzone (22) des Deckels (4) befestigt ist, derart, dass das Gehäuse (1) hermetisch verschlossen wird unter Einhüllen des Behälters (2) und nicht das Kulturmilieu (14) aufdeckt, wenn das Verschlussorgan (24) weggenommen ist.

2. Box nach Anspruch 1, **dadurch gekennzeichnet, dass** das Verschlussorgan (24) durchsichtig ist.

3. Box nach Anspruch 1 oder 2, **dadurch gekennzeichnet, dass** der Deckel (4) einen biegsamen Rand (18) aufweist, der die Seitenwände (8) des Behälters (2) umgibt, wobei dieser Rand (18) verformt werden kann, um die Seitenwände (8) des Behälters zu klemmen.

4. Box nach Anspruch 3, **dadurch gekennzeichnet, dass** der biegsame Rand (18) einen vorspringenden Abschnitt (26) aufweist, der in der Lage ist, mit einem komplementären Abschnitt des Behälters (2) zusammenzuwirken, wenn der Rand (18) verformt ist, derart, dass er den Deckel (4) auf dem Behälter (2) hält.

5. Box nach Anspruch 3 oder 4, **dadurch gekennzeichnet, dass** der Behälter (2) einen Rand (10, 12) in Form eines umgedrehten L aufweist, der wenigstens teilweise die Seitenwände (8) des Behälters umgibt, wobei die biegsame Seitenwand (18) des Deckels (4) in der Lage ist, den L-förmigen Rand (10, 12) des Behälters zu klemmen, ohne eine Verformung der Seitenwände (8) des Behälters (2) hervorzurufen.

6. Box nach einem der Ansprüche 1 bis 5, **dadurch gekennzeichnet, dass** sie Gelenkeinrichtungen des Deckels (4) an dem Behälter (2) aufweist, derart, dass sie die Öffnung und das Schließen im Raum der Box (1) mit nur einer Hand ermöglicht.

7. Box nach Anspruch 6, **dadurch gekennzeichnet, dass** die Gelenkeinrichtungen durch wenigstens eine Zapfen (40), der an dem Deckel (4) vorgesehen ist, und durch wenigstens eine komplementäre Nut (34) gebildet sind, die in dem Behälter (2) vorgesehen ist, eine Nut (34) in die der Zapfen eingreift, was ebenfalls ermöglicht, die Box durch eine vertikale Bewegung des Deckels zu öffnen oder zu schließen.

8. Box nach Anspruch 7, **dadurch gekennzeichnet, dass** der Behälter (2) einen äußeren Kranz (32) aufweist, und dass der Deckel (4) eine innere Seitenwand (36) und eine äußere Seitenwand (38) aufweist, wobei der Zapfen (40) an der inneren Seitenwand (36) des Deckels (4) und die Nut (34) in dem Kranz (32) des Behälters (2) vorgesehen ist.

9. Box nach Anspruch 7 oder 8, **dadurch gekennzeichnet, dass** die Nut (34) des Kranzes (32) des Behälters (2) zwei Anschlagoberflächen (46, 48) aufweist, um die Öffnung des Deckels (4) zu begrenzen.

10. Box nach einem der Ansprüche 6 bis 9, **dadurch gekennzeichnet, dass** der Deckel (4) mit einem Funktionszapfen (42) versehen ist, der seine Öffnung erleichtert.

11. Boxanordnung (1), **dadurch gekennzeichnet, dass** sie aus einem Band (60) und einer Reihe von Boxen (1) nach einem der Ansprüche 1 bis 10 gebildet ist, die nacheinander auf dem Band (60) angeordnet sind, wobei jede Box (1) auf dem Band (60) durch ihre Flanschzone (22) befestigt ist, wobei das Band (60) die Rolle eines Verschlussorgans (24) für jedes Gehäuse (1) spielt.

12. Boxenanordnung nach Anspruch 11, **dadurch gekennzeichnet, dass** die Boxen (1) auf nur einer Seite des Bandes (60) angeordnet sind.

13. Boxenanordnung nach Anspruch 11, **dadurch gekennzeichnet, dass** die Boxen (1) abwechselnd auf der einen und der anderen Seite des Bandes (60) angeordnet sind.

14. Verteiler für die Verteilung von Boxen nach einem der Ansprüche 1 bis 10, wobei die Boxen Teil einer Boxenanordnung nach einem der Ansprüche 11 bis 13 sind, **dadurch gekennzeichnet, dass** er ein Speichermagazin (86) des Bandes (60); eine Antriebseinrichtung (88) des Bandes; eine Trenneinrichtung (92) um eine Box (1) von dem Band (60) zu trennen; wenigstens eine Aufnahmeplatte (94) der Boxen (1), nachdem sie von dem Band (60) getrennt wurden, aufweist.

15. Verteiler nach Anspruch 14, **dadurch gekennzeichnet, dass** das Band gemäß dem Anspruch 13 ist, dass es eine Führung (96) aufweist, die die aufeinanderfolgenden Boxen (1) des Bandes (60) aufweist, mit einem Neigungswinkel bezüglich der Bewegungsrichtung des Bandes (60) und dass die Führung (96) zwischen zwei geneigten Positionen symmetrisch bezüglich der Bewegungsrichtung des Bandes (60) hin und her schwingt, wobei die Führung (96) die eine oder die andere dieser Positionen einnimmt, in Abhängigkeit von der Seite des Bandes auf der die Box befestigt ist.
